# EUROPEAN PATENT SPECIFICATION

(11) **EP 1 235 535 B1**
(45) Date of publication and mention of the grant of the patent: **19.09.2007**
(21) Application number: 00983514.1
(22) Date of filing: 06.12.2000
(51) Int. Cl.: A61F 2/90, A61L 27/00, A61M 29/00

(54) **STENT**
STENT
ENDOPROTHESE VASCULAIRE

(30) Priority: 07.12.1999 KR 9955580
(43) Date of publication of application: 04.09.2002
(73) Proprietor: Sewoonmedical Co., Ltd., Seoul 130-070 (KR)
(72) Inventor: LEE, Gil-Whan, Seoul 143-224 (KR); LEE, Gwui-Mun, Hwasung-kun, Kyungki-do 445-970 (KR); KIM, Sang-Chul, Suwon, Kyungki-do 441-460 (KR)
(74) Representative: Wakerley, Helen Rachael
(86) International application number: PCT/KR2000/001412
(87) International publication number: WO 2001/041673

(56) References cited:
- EP-A1- 0 928 604
- EP-A2- 0 686 379
- WO-A2-96/19952
- US-A- 5 667 523
- US-A- 5 720 776

## Description

### BACKGROUND OF THE INVENTION

### (a) Field of the Invention

The present invention relates to a stent and, more specifically, to a stent which can be manufactured densely in a simple way with a minimized processing time and which is configured to be pliable according to the crooked configuration of a body lumen without refraction so that it has an enhanced partial expansion force at the curved portions and is not readily movable at the inserted position in the body lumen.

### (b) Description of the Related Art

It generally appears that a narrowed lumen due to, for example, esophagostenosis from cancer of the esophagus or arteriosclerosis results in poor circulation of blood, In such a case, a stent is inserted into the narrowed lumen as a means for expanding the narrowed passage of the lumen and maintaining the expanded position for the purpose of improved circulation of the blood or articles of diet.

The stent generally has a self-elastic cylindrical structure that is constricted under external forces and takes an expanded position by itself when it is left in an unloaded condition free of the external forces.

In order to function properly, those stents that take an expanded position by themselves have to possess certain properties, for example, the ability to expand the lumen with a force, enabling successful flexible implantation according to the crooked configuration of the body lumen and the ability to remain fixed at the inserted position in the body lumen.

An exemplary self-expandable stent is of a wall type that is disclosed in U.S. Patent No. 4,655,771.

The wall-type stent comprises a flexible tubular body which has a diameter that is variable by axial movement of the ends of the body relative to each other and which is composed of several individual rigid but flexible thread elements each of which extends in a helix configuration with the central line of the body as a common axis. The body comprises first elements having the same direction of winding but being axially displaced relative to each other, and second elements crossing the first elements. The second elements are axially displaced relative to each other but have an opposite direction of winding.

Another exemplary self-expandable stent is of a helix-type that is provided with filaments having a unit length and which wind around the cylindrical wall surface of the stent in a helix configuration. The filaments reciprocate in a zigzag fashion between both ends of the stent and cross each other to form a cylindrical mesh structure.

### SUMMARY OF THE INVENTION

In regard to the wall-type stent, which is composed of thread elements each extending in a helix configuration with the central line of the body as a common axis and reciprocating between both ends of the body, the manufacturing performance deteriorates with a longer manufacturing time and the productivity lowers as the denseness of the stent increases.

The wall-type stent is flexible so as to be constricted in a very small diameter and readily bent at the curved portions of the body lumen, but it is greatly deteriorated because of the partial expansion force at the curved portions according to the crooked configuration of the body lumen, and is readily moved from the inserted position in the body lumen.

Meanwhile, the helix-type stent is also excellent in flexibility so as to be constricted in a very small diameter, but due to such good flexibility, is readily movable from the implanted position in the body lumen and it is hard to manufacture densely- Furthermore, the helix-type stent has a great deterioration because of the partial expansion force at the curved portions according to the crooked configuration of the body lumen, so that much refraction appears at the curved portions.

It is therefore an object of the present invention to solve the problems and to provide a stent which can be manufactured densely in a simple way in order to minimize processing time and which is configured to be pliable according to the crooked configuration of a body lumen without refraction so that it has an enhanced partial expansion force at the curved portions and is not readily movable at the inserted position in the body lumen.

US-A-5667523 discloses a stent having a repeated pattern of two circumferential nonoverlapping zigzag wires which are mirror symmetrical on each side of a circumference.

A stent according to the invention is defined in claim 1.

### BRIEF DESCRIPTION OF THE DRAWINGS

The accompanying drawings, which are incorporated in and constitute a part of the specification, illustrate an embodiment of the invention, and, together with the description, serve to explain the principles of the invention:
FIG. 1 is a front view of a stent according to a first embodiment of the present invention:
FIG. 2 is a development diagram of the stent according to the first embodiment of the present invention, in which first unit members make two turns;
FIG. 3 is a development diagram of the stent according to the first embodiment of the present invention, in which second unit members make two turns;
FIG. 4 is a development diagram of the stent according to the first embodiment of the present invention, in which the first and second unit members make several turns and combine together; and
FIG. 5 is a front view showing a stent according to a second embodiment of the present invention.

### DETAILED DESCRIPTION OF THE PREFERRED EMBODIMENTS

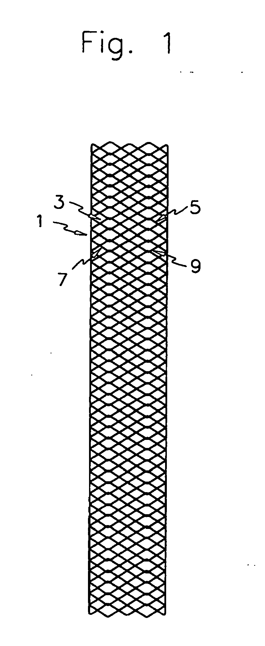

Referring to FIGS. 1 to 4, a stent 1 according to a first embodiment of the present invention comprises first and second unit members each of which have a unit length and flexibly expand by themselves.

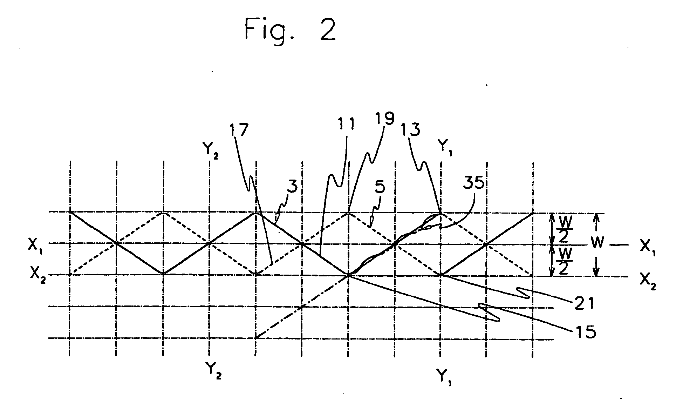

The first unit members are longitudinally arranged at. regular intervals with a predetermined width apart along a base circumference so as to form a cylindrical structure and make a first turn 3 (Fig 2) (a first one-turn)-in a zigzag fashion in a predetermined cycle on the basis of a reference point (X1-X1, Y1-Y1).

Following the first turn 3, as shown in Fig. 2, the first unit members make a second turn 5 (a second one-turn) in a zigzag fashion and cross the unit members of the first turn 3 with a phase difference between the bending points of the first and second turns 3 and 5 thereby forming a mesh.

The second unit members are longitudinally arranged at regular intervals In a predetermined width along the same circumference as the first unit members so as to form a cylindrical structure. The second unit members have a reference point (X2-X2, Y2-Y2) spaced at a predetermined distance from a point opposing the reference point of the first turn 3 of the first members in the longitudinal direction. The second unit members regularly cross each other between the first turn 3 and the second turn 5 of the first members with a phase difference from the first turn 3 of the first members and make a first tum 7 (a first two-tum) in a zigzag fashion.

Following the first turn 7 the second unit members cross each other in correspondence to the bending points of the first turn 7 with a phase difference from the first turn 7 of the second unit members to form a mesh structure and regularly cross each other at positions between the first turn 3 and the second turn 5 of the first unit members to form a mesh structure. The second unit members thus make a second turn 9 (a second two-tum) in a zigzag fashion.

Preferably, the first and second turns 3 and 5 of the first unit members and the first and second turns 7 and 9 of the second unit members have the same axial width W based on the central lines X1-X1 and X2-X2 each having a width difference W/2, and the same cycle with a phase difference so that they are flexibly adapted to the crooked configuration of the body lumen.

The first turn 3 of the first members comprises a plurality of straight lines 11 defined by a plurality of bending points formed by a plurality of first peaks 13 and first valleys 15 which interconnect the first straight lines 11.

The second turn 5 of the first members comprises a plurality of straight lines 17 which are defined by a plurality of-bending points and cross the straight_lines 11 to form a mesh, forming a plurality of peaks 19 and valleys 21 which interconnect the straight lines 17 and correspond to the valleys 15 and the peaks 13.

The first tum 7 of the second members comprises a plurality of straight lines 23 which are defined by a plurality of bending points and cross the straight lines 17 to form a mesh, and plurality of peaks 25 and valleys 27 which interconnect the straight lines 23.

The second tum 9 of the second members comprises a plurality of straight lines 29 which are defined by a plurality of bending points and cross the straight lines 11 and 17 of the first members and the straight lines 23 to form a mesh, and a plurality of peaks 31 and valleys 33 which interconnect the straight lines 29 and correspond to the valleys 27 and the peaks 33.

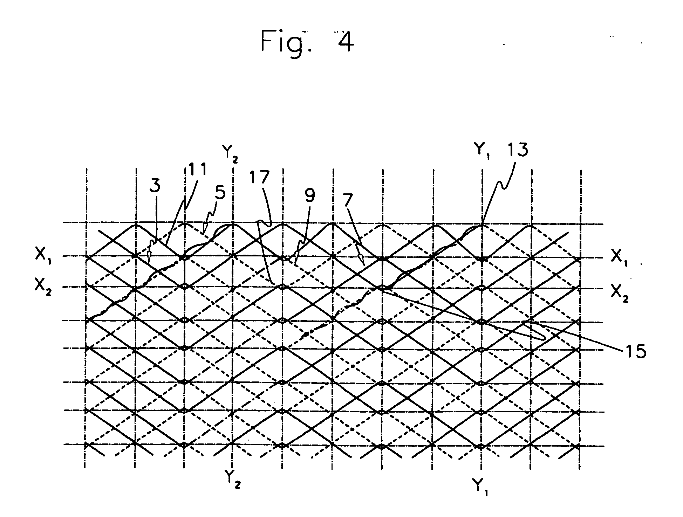

The first unit members, after forming their second turn 5 and prior to the first turn 7 of the second members form a first connection 35 having at least one twist along one of the straight lines 11 of the first turn 3 and then extend in a helix configuration towards one of the straight lines 11 of the next first turn 3, as shown in FIG. 4.

The second unit members, after forming their second tum 9 and prior to the next turn 3 neighbouring the second turn 9, form a second connection 37 having at least one twist along one of the second straight lines 23 of the tum 7 of the second members and then extend in a helix configuration towards the lines 23 of the next first tum 7 of the second members.

The peaks 13 and the valleys 15 of the first turns 3 of the first members are fixedly interengaged with the corresponding valleys 15 and peaks 13 of the next turn 3, as shown in FIG. 4. And each peak 19 and each valley 21 of a second turn 5 are fixedly interengaged with the corresponding valley 21 and peak 19 of the next second turn 5.

Each peak 25 and each valley 27 of a first turn 7 of the second members are fixedly interengaged with the corresponding valley 27 and peak 25 of the next first turn 7. Each peak 31 and valley 33 of the second turn 9 of the second member is fixedly interengaged with the corresponding valley 33 and peak 31 of the next second turn 9 of the second members.

Preferably, the first and second unit members are formed from a shape memory alloy. for example, nitinol so that they have an expansion force at a temperature approximate to the temperature of the body lumen.

In the stent 1 according to the first embodiment of the present invention. the first and second turns 3 and 5 of the first unit members and the first and second turns 7 and 9 of the second unit members cross each other with a predetermined width difference and a predetermined phase difference and interferingly make a tum in a zigzag fashion, while the straight lines 11, and straight lines 17 the first unit members and, the straight lines 23 and 29 cross each other to form a mesh structure.

Therefore, the stent 1 has very small spaces between the first one-turn 3 and the first two-tum 5 and between the second one-turn 7 and the second two-turn 9 and can be manufactured in a reduced time in a simple way.

The peaks 13, and 19, and the valleys 15 and 21 are, as shown in FIG. 4, fixedly interconnected with valleys 15, and 21. and the peaks 13 and 19 neighboring first and second turns 3 and 5 respectively.

Thus, the first and second turns 3 and 5 of the first unit members and the first and second turns 7 and 9 of the second unit members are fixedly interconnected with each other at the respective connections and simply crossed at the respective crossings. The connections and the crossings are alternatively positioned along the one circumference of the stent 1 and arranged at regular intervals on the circumference to form a straight line-like configuration.

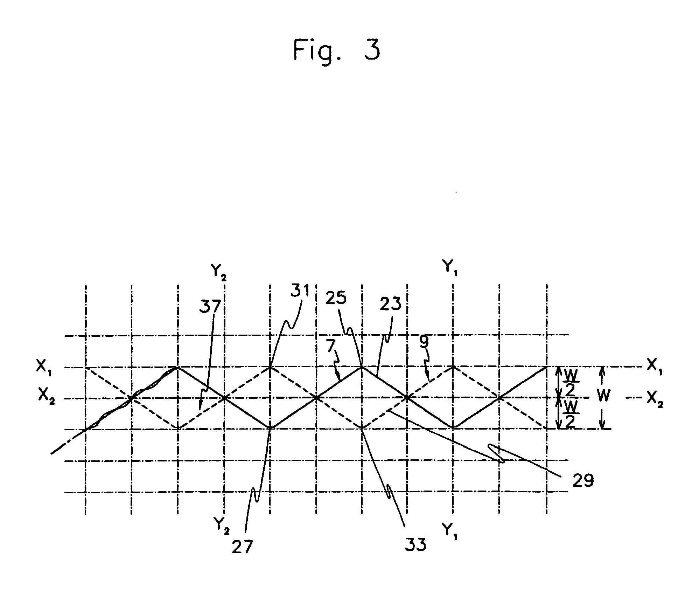

The peaks 25, and 31, and the valleys 27 and 33 (FIG. 3) are fixedly interconnected with the valleys 27, and 33, and peaks 25 and 31 neighboring the first and second turns 7 and 9, respectively.

The first unit members form connections having at least one twist along straight lines 11 of the first 3 after forming the second turn 5, and the second unit members form connections having at least one twist along straight lines 23 of the first tum 7 of the second unit members.

Therefore, the stent 1 has such a good expanding property that it can be readily inserted into a narrowed region of the body lumen and it maintains a constant curvature in providing a passage for the body lumen, which prevents refraction and maintains the shape, for example, a cylindrical configuration even when making a sharp curve along the various configurations of the body lumen.

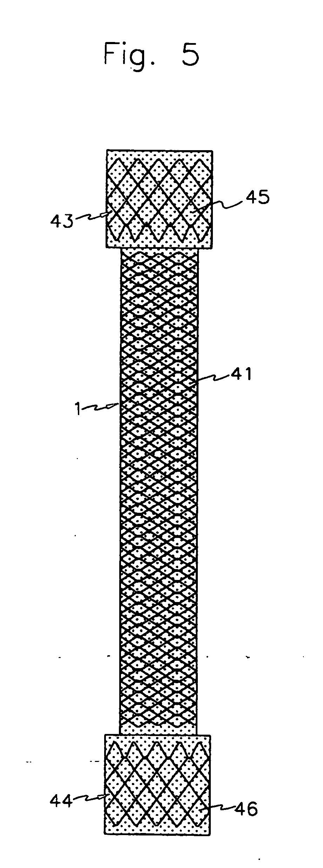

FIG. 5 is a diagram showing a second embodiment of the present invention, which is similar to the first embodiment except for the following features.

The stent 1 according to the second embodiment of the present invention is coated with a coating 41, for example, polyurethane, which covers the inner and outer surfaces of the stent 1 to minimize friction between the stent 1 and the body lumen when the stent 1 is expanded in the narrowed region of the body lumen and to allow the blood or articles of diet to readily pass through the body lumen.

The coating 41 is warmed at a predetermined temperature and applied to the outer surface of the stent 1. Alternatively, the stent 1 is immersed in a tank containing the coating 41 and then taken out to cool the coating.

The stent 1 has either end or both ends thereof provided with the movement - preventing members 43 and 44, which have a larger diameter than the stent 1 and support the stent 1 being inserted into the narrowed region of the body lumen to prevent the stent 1 from moving in the body lumen.

The movement preventing members 43 and 44 can be of any structure that is wound in the same manner as the stent 1, or composed of unit members wound in a zigzag fashion in a helix configuration. The movement preventing members 43 and 44 together with the stent 1 are integrally provided with coatings 45 and 46 at the inner and outer surfaces.

The movement preventing members 43 and 44 have a center common to the stent 1 so that they are readily expandable when inserted into the narrowed region of the body lumen.

As described above, the stent of the present invention can be manufactured densely in a simple way for a minimized processing time to enhance productivity and reduce the manufacturing time.

Also, the stent of the present invention is configured to be pliable according to the crooked configuration of a body lumen without refraction so that it has an enhanced partial expansion force at the curved portions and is not readily movable at the inserted position in the body lumen. Thus the present invention successfully provides a passage for the bending narrow portion of the body lumen and allows the blood or articles of diet to readily pass through the lumen.

Furthermore, the stent of FIG. 5 is provided with a coating to secure good circulation of the blood or articles of diet and prevent the blood or article of diet from entering other organs.

## Claims

1. A generally cylindrical stent (1) comprising a first mesh unit and a second mesh unit, in which filaments of the first mesh unit make a first turn (3) in a zigzag fashion in a cycle with a first reference point (X₁. Y1) and also make a second tum (5) in a zigzag fashion overlying the first turn (3) with a phase difference (15. 21) therefrom to form a mesh portion,
in which filaments of the second mesh unit make a first turn (7) in a zigzag fashion in a cycle with a second reference point (X2. Y2) opposing the first reference point (X1, Y1) and also make a second tum (9) in a zigzag fashion overlying the first turn (7) of the second mesh unit with a phase difference therefrom to form a further mesh portion,
and in which the filaments of the second mesh unit regularly cross each other at positions between the filaments of the first mesh unit, the first and second reference points (X1, Y1; X2, Y2) being axially spaced apart (W/2).

2. The stent as claimed in claim 1, wherein the first turn (3) and the second turn (5) of the first unit comprise a plurality of straight lines (11, 17) defined by a plurality of bending points formed by a plurality of first and second peaks (13, 19) and first and second valleys (15, 21) each of which interconnects the first straight lines (11, 17) respectively,
wherein the first turn (7) and the second turn (9) of the second unit comprise a plurality of second straight lines (23, 29) defined by a plurality of bending points formed by a plurality of peaks (25, 31) and second valleys (27, 33) each of which interconnects the straight lines (23, 29) of the second unit, wherein the straight lines (23) cross the straight lines (29) to form a mesh, and the second straight lines (23) and the second straight lines (29) cross the straight lines (11, 17) of the first unit to form a mesh.

3. The stent as claimed in claim 2, wherein the filaments of the first unit members provide a connection (35) having at least one twist along a straight line (11) of the first turn after forming the second turn (5) and prior to forming the next first turn, and
wherein the filaments of the second unit members provide a connection having at least one twist along a straight line of the first turn (7) after forming the second turn (9) and prior to the next first turn (7) neighboring the said second turn (9).

4. The stent as claimed in claim 2 or 3 wherein the first peak (13) the second peak (19) the first valley (15) and the second valley (21) of the first tum of the first unit and the second turn of the first unit are fixedly interconnected respectively with the first valley (15). the second valley (21), the first peak (13) and the second peak (19) of the first turn (3) and the second tum (5) neighbouring the first turn (3) and second turn (5), and wherein the first peak (25), the second peak (31), the first valley (27) and the second valley (33) of the first turn (7) and the second turn (9) of the second unit are fixedly interconnected with the valley (27), the valley (33). the first peak (25) and second peak (31) of the first turn (7) and the second turn (9) neighboring the first turn (7) and second turn (9) respectively.

5. The stent as claimed in any preceding claim, wherein the stent is coated with a coating for covering inner and outer surfaces of the stent to minimize friction with a body lumen when expanded in the narrowed region of the body lumen and to allow the blood or articles of diet to readily pass through the lumen.

6. The stent is claimed in any preceding claim, wherein the stent has either and or both ends thereof provided with a movement-preventing member (43, 44), the movement-preventing member having a larger diameter than the remainder of the stent and supporting the stent when inserted into the narrowed region of the body lumen to prevent the stent from moving in the body lumen.

## Patentansprüche

1. Stent (1), der allgemein zylindrisch ist und Folgendes umfasst: eine erste Netzeinheit und eine zweite Netzeinheit, wobei Filamente der ersten Netzeinheit eine erste Drehung (3) in Zickzackweise in einem Zyklus mit einem ersten Referenzpunkt (X1, Y1) macht und außerdem eine zweite Drehung (5) in Zickzackweise über der ersten Drehung (3) liegend mit einer Phasendifferenz (15, 21) dazu zur Bildung eines Netzabschnitts macht,
wobei Filamente der zweiten Netzeinheit eine erste Drehung (7) in Zickzackweise in einem Zyklus mit einem dem ersten Referenzpunkt (X1, Y1) gegenüberliegenden zweiten Referenzpunkt (X2, Y2) macht und außerdem eine zweite Drehung (9) in Zickzackweise über der ersten Drehung (7) der zweiten Netzeinheit liegend mit einer Phasendifferenz dazu zur Bildung eines weiteren Netzabschnitts macht,
und wobei die Filamente der zweiten Netzeinheit einander regelmäßig an Positionen zwischen den Filamenten der ersten Netzeinheit kreuzen, wobei der erste und der zweite Referenzpunkt (X1, Y1; X2, Y2) axial voneinander beabstandet sind (W/2).

2. Stent nach Anspruch 1, wobei die erste Drehung (3) und die zweite Drehung (5) der ersten Einheit mehrere gerade Linien (11, 17) umfassen, die durch mehrere Krümmungspunkte definiert werden, die durch mehrere erste und zweite Gipfel (13, 19) und erste und zweite Täler (15, 21) gebildet werden, die jeweils die ersten geraden Linien (11, 17) miteinander verbinden,
wobei die erste Drehung (7) und die zweite Drehung (9) der zweiten Einheit mehrere zweite gerade Linien (23, 29) umfassen, die durch mehrere Krümmungspunkte definiert werden, die durch mehrere Gipfel (25, 31) und zweite Täler (27, 33) gebildet werden, die jeweils die geraden Linien (23, 29) der zweiten Einheit miteinander verbinden, wobei die geraden Linien (23) die geraden Linien (29) zur Bildung eines Netzes kreuzen und die zweiten geraden Linien (23) und die zweiten geraden Linien (29) die geraden Linien (11, 17) der ersten Einheit zur Bildung eines Netzes kreuzen.

3. Stent nach Anspruch 2, wobei die Filamente der Elemente der ersten Einheit eine Verbindung (35) bereitstellen, die wenigstens eine Verdrehung entlang einer geraden Linie (11) der ersten Drehung nach dem Bilden der zweiten Drehung (5) und vor dem Bilden der nächsten ersten Drehung aufweist, und
wobei die Filamente der Elemente der zweiten Einheit eine Verbindung bereitstellen, die wenigstens eine Verdrehung entlang einer geraden Linie der ersten Drehung (7) nach dem Bilden der zweiten Drehung (9) und vor dem Bilden der nächsten ersten Drehung (7) aufweist, die der genannten zweiten Drehung (9) benachbart ist.

4. Stent nach Anspruch 2 oder 3, wobei der erste Gipfel (13), der zweite Gipfel (19), das erste Tal (15) und das zweite Tal (21) der ersten Drehung der ersten Einheit und der zweiten Drehung der ersten Einheit jeweils fest mit dem ersten Tal (15), dem zweiten Tal (21), dem ersten Gipfel (13) und dem zweiten Gipfel (19) der ersten Drehung (3) und der zweiten Drehung (5) verbunden sind, die der ersten Drehung (3) und der zweiten Drehung (5) benachbart sind, und wobei der erste Gipfel (25), der zweite Gipfel (31), das erste Tal (27) und das zweite Tal (33) der ersten Drehung (7) und der zweiten Drehung (9) der zweiten Einheit fest mit dem Tal (27), dem Tal (33), dem ersten Gipfel (25) und dem zweiten Gipfel (31) der ersten Drehung (7) und der zweiten Drehung (9) verbunden sind, die jeweils der ersten Drehung (7) und der zweiten Drehung (9) benachbart sind.

5. Stent nach einem der vorherigen Ansprüche, wobei der Stent mit einem Überzug zum Bedecken der Innen- und Außenfläche des Stents beschichtet ist, um Reibung mit einem Körperlumen zu minimieren, wenn er in der verengten Region des Körperlumens expandiert ist, und um Blut oder Nahrungsstücke problemlos durch das Lumen passieren zu lassen.

6. Stent nach einem der vorherigen Ansprüche, wobei der Stent an einem und/oder an beiden seiner Enden mit einem Element (43, 44) zum Verhindern einer Bewegung versehen ist, wobei das Bewegungsverhinderungselement einen größeren Durchmesser als der Rest des Stents hat und den Stent unterstützt, wenn er in die verengte Region des Körperlumens eingeführt ist, um zu verhindern, dass sich der Stent in dem Körperlumen bewegt.

## Revendications

1. Stent généralement cylindrique (1) comprenant une première unité de maille et une deuxième unité de maille, dans lequel des filaments de la première unité de maille font un premier tour (3) en zigzag dans un cycle avec un premier point de référence (X1, Y1) et font aussi un deuxième tour (5) en zigzag recouvrant le premier tour (3) avec une différence de phase (15, 21) de celui-ci pour former une partie de maille.
dans lequel des filaments de la deuxième unité de maille font un premier tour (7) en zigzag dans un cycle avec un deuxième point de référence (X2, Y2) à l'opposé du premier point de référence (X1, Y1) et font aussi un deuxième tour (9) en zigzag recouvrant le premier tour (7) de la deuxième unité de maille avec une différence de phase de celui-ci pour former une autre portion de maille,
et dans lequel les filaments de la deuxième unité de maille se croisent régulièrement les uns les autres à des positions entre les filaments de la première unité de maille, le premier et le deuxième points de référence (X1, Y1:X2, Y2) étant espacés sur le plan axial (W/2).

2. Le stent tel que revendiqué dans la revendication 1, dans lequel le premier tour (3) et le deuxième tour (5) de la première unité comprennent une pluralité de lignes droites (11, 17) définies par une pluralité de points de pliage formés par une pluralité de premières et de deuxièmes crêtes (13, 19) et de premiers et deuxièmes creux (15, 21) chacun desquels interconnecte respectivement les premières lignes droites (11, 17),
dans lequel le premier tour (7) et le deuxième tour (9) de la deuxième unité comprennent une pluralité de deuxièmes lignes droites (23, 29) définies par une pluralité de points de pliage formés par une pluralité de crêtes (25, 31) et de deuxièmes creux (27, 33) chacun desquels interconnecte les lignes droites (23, 29) de la deuxième unité, où les lignes droites (23) croisent les lignes droites (29) pour former une maille, et les deuxièmes lignes droites (23) et les deuxièmes lignes droites (29) croisent les lignes droites (11, 17) de la première unité pour former une maille.

3. Le stent tel que revendiqué dans la revendication 2, dans lequel les filaments des membres de la première unité fournissent une connexion (35) ayant au moins une torsion le long d'une ligne droite (11) du premier tour après avoir formé le deuxième tour (5) et avant de former le premier tour suivant, et
dans lequel les filaments des membres de la deuxième unité fournissent une connexion ayant au moins une torsion le long d'une ligne droite du premier tour (7) après avoir formé le deuxième tour (9) et avant le premier tour suivant (7) avoisinant ledit deuxième tour (9).

4. Le stent tel que revendiqué dans la revendication 2 ou 3, dans lequel la première crête (13), la deuxième crête (19), le premier creux (15) et le deuxième creux (21) du premier tour de la première unité et du deuxième tour de la première unité, sont interconnectés de manière fixe respectivement avec le premier creux (15), le deuxième creux (21), la première crête (13) et la deuxième crête (19) du premier tour (3) et du deuxième tour (5) avoisinant le premier tour (3) et le deuxième tour (5), et dans lequel la première crête (25), la deuxième crête (31), le premier creux (27) et le deuxième creux (33) du premier tour (7) et du deuxième tour (9) de la deuxième unité sont interconnectés de manière fixe avec le creux (27), le creux (33), la première crête (25) et la deuxième crête (31) du premier tour (7) et du deuxième tour (9) avoisinant le premier tour (7) et le deuxième tour (9) respectivement.

5. Le stent tel que revendiqué dans l'une quelconque des revendications précédentes, où le stent est enrobé d'un enrobage pour couvrir les surfaces internes et externes du stent afin de minimiser la friction avec un passage corporel lorsqu'il est en expansion dans la région rétrécie du passage corporel et pour permettre au sang ou à des articles de nourriture de passer facilement à travers le passage.

6. Le stent tel que revendiqué dans l'une quelconque des revendications précédentes, où le stent a une ou ses deux extrémités dotées d'un membre de prévention de mouvement (43, 44), le membre de prévention de mouvement ayant un diamètre plus grand que le reste du stent et supportant le stent lorsque introduit dans la région rétrécie du passage corporel afin d'empêcher le stent de bouger dans le passage corporel.
